(19) Europäisches Patentamt
European Patent Office
Office européen des brevets

(11) **EP 3 466 322 A1**

(12) **EUROPEAN PATENT APPLICATION**

(43) Date of publication:
**10.04.2019 Bulletin 2019/15**

(51) Int Cl.:
*A61B 5/00* (2006.01)    *A61B 5/053* (2006.01)
*A61B 5/16* (2006.01)

(21) Application number: **17382661.1**

(22) Date of filing: **04.10.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Fundación Instituto de Robótica para la Dependencia**
**08870 Sitges (ES)**
• **Universitat Rovira I Virgili**
**43003 Tarragona (ES)**

(72) Inventors:
• **SHUKLA, Jainendra**
**08870 Sitges (ES)**
• **OLIVER, Joan**
**08014 Barcelona (ES)**
• **PUIG VALLS, Domènec Savi**
**43007 Tarragona (ES)**

(74) Representative: **Juncosa Miró, Jaime et al Torner, Juncosa i Associats, S.L.**
**Gran Via de les Corts Catalanes, 669 bis, 1o, 2a**
**08013 Barcelona (ES)**

(54) **A COMPUTER-IMPLEMENTED METHOD FOR THE MEASUREMENT OF HUMAN EMOTION OF A SUBJECT AND A METHOD FOR FILTERING AN EDA SIGNAL**

(57)    The method comprises: filtering an EDA signal via wavelet and further modelling detail coefficients of said wavelet using a Laplace distribution, obtaining a denoised EDA signal; normalizing said denoised EDA signal using baseline values of the subject's EDA signal; calculating a set of feature values from the normalized EDA signal; and classifying a subject's arousal and/or stress level as a component of a human emotion based on the calculated set of feature values.

Fig. 1

EP 3 466 322 A1

**Description**

Field of the invention

The present invention relates to a computer-implemented method for the measurement of human emotion of a subject and to a method for filtering an Electrodermal Activity (EDA) signal, preferably in online applications, i.e. in real time. To achieve the object the present invention mainly relates to monitoring of EDA.

According to present invention, arousal is the intensity of the emotional activation of a subject or user, ranging from excited to calm. Valence is orthogonal to the arousal and is the direction of the behavioral activation associated with emotion, either toward (appetitive motivation, pleasant emotion) or away from (aversive motivation, unpleasant emotion) a stimulus.

Background of the invention

Electrodermal activity (EDA) refers to the changes in conductivity in the skin, which is affected by the activity of the sympathetic branch of the autonomous nervous system and is usually considered as a correlate of psychological processes such as emotional arousal, stress, or cognitive effort. It is commonly measured by placing two electrodes in palmar sites of the hand, although also other alternative locations, such as the wrist, have been also proposed.

Due to its low-cost and easy-to-collect nature, EDA measurement has been commonly used in research in psychology and as a tool for the assessment of user's experience in a variety of contexts such as recreational and serious games, driving, visual discomfort with 3D media, internet browsing, or patient-robot interaction.

The availability of computing devices, e.g. wearable, able to measure online EDA provide the opportunity for real world data collection in a comfortable manner in real-life scenarios outside the lab. This allows broadening the scope of EDA analysis from simply gathering information on user's psychological state to the online adaptation of the system according to user's cognitive and emotional states. The advantages of such online adaptation are especially remarkable in the cases of users with acute problems to identify, report, and regulate their emotions, such as children or individuals with intellectual developmental disabilities (IDD). In this sense, novel cognitive stimulation systems based on robots or virtual reality may greatly benefit from an online input of the user emotional state from EDA signal.

However, compared to laboratory studies, the online collection and analysis of EDA signals in real-life context involves new challenges related to signal processing. While in lab experiments the participants are usually asked to not to move the hand to which the electrodes are attached, hand's movement can be hardly controlled in real-life settings. As a consequence of such movement, partial detachment of the electrodes or pressure over them may occur, leading to the appearance of artifacts in the signal. If these artifacts remain in the signal when it is analyzed they can easily be misinterpreted and skew the analysis; for example, they may be mistaken for a skin conductance response (SCR) (a physiological reaction that may indicate increased stress), especially when the analysis is conducted automatically. Even in a context in which not much movement should be expected, the presence of such motion artifacts can be critical in the case of users whose ability to control the level of movement is limited (e.g. individuals with IDD).

Methods used in previous research to correct artifacts mainly consist of exponential smoothing [1] and low-pass filtering [2]. Over the last two decades, wavelets have already proven their significant value in signal processing and image. Wavelets have also been found suitable for EDA activity modeling, given its nonstationary behavior. Wavelet based sophisticated de-noising of motion artifacts have been widely used in researches [3, 4, 5, 6] and has offered better results due to the good localization property of the wavelet transforms [4]. On the other hand, other sophisticated methods, not based in wavelets, have also been proposed. Recently, [7] proposed a deconvolution based approach (called cvxEDA) using Maximum a Posteriori (MAP) estimation and convex optimization which provided a decomposition of the EDA that is robust to noise.

However, the aforementioned techniques present several important limitations when used for online filtering of the EDA signals, such as the following:

- Exponential smoothing [1] and low pass filtering based denoising [2] methods are not able to atone the unexpectedly occurring artifacts which have higher values than EDA and undiscriminated filtering of the whole signal also distorts the EDA signals without artifacts [6].
- Denoising with the traditional DWT wavelet transform [5] can exhibit visual artifacts due to lack of translation invariance and "pseudo-Gibbs" oscillations are especially pronounced in the vicinity of discontinuities.
- Estimation of the noise level $\sigma$ as proposed by [5] is based on the data collected during the rest period. This type of noise level estimation is an offline and static measure and is an overhead cost on denoising. Moreover, as the actual nature of noise in the noisy signal is dynamic, hence the noise level estimation needs to be done online.
- Gaussian mixture based modeling for the distribution of the wavelet coefficients [6] requires estimation of three model parameters, the mixture parameter and the variances of the two Gaussians which demand employment of iterative algorithms such as Expectation Maximization (EM) algorithm. These algorithms have high computational complexity.

Whereas in the offline analysis of EDA signals visual inspection allows identifying and remove the parts of the recording containing artifacts, online adaptation of systems based on EDA signal requires automated methods for such purpose. These methods not only need to be accurate enough to provide a good signal quality, but also need to be computationally affordable enough to work online.

More solutions are therefore needed for filtering motion artifacts in EDA signals to provide actual emotional state and engagement level of users while minimizing distortions and lowering computational costs and this invention aims to provide a solution to this challenge.

Description of the Invention

Aspects of the present invention provide a computer-implemented method for the measurement of human emotion of a subject.

In an embodiment of this invention an EDA signal is obtained in response to one or more stimulus presented to a subject during a given period of time via a measuring device including a wearable or non-wearable device such as Shimmer GSR+, Feel Wristband, Embrace Wristband, E4 Wristband, BIOPAC EDA100C-MRI, among others. The method in a characteristic manner comprises filtering the obtained EDA signal via wavelet and further modelling detail coefficients of said wavelet using a Laplace distribution, obtaining a denoised EDA signal; normalizing the denoised EDA signal using baseline values of the subject's EDA signal; calculating a set of feature values from the normalized EDA signal; and classifying a subject's arousal and/or stress level as a component of a human emotion based on the calculated set of feature values.

The proposed method is performed, preferably, online. So the component of human emotion can be directly obtained during the interaction of the subject with information and communication applications, allowing a quick processing and reaction from the side receiving the information

In an embodiment, the wavelet is a Stationary Wavelet Transform (SWT). In this case the level of decomposition $J$ is set to ($log_2 freq + 2$), where $freq$ is the frequency of the signal. Preferably, the $haar$ wavelet is used as mother wavelet. Alternatively, other wavelets could be used as mother wavelets, for instance the $db3$ and the $coiflet3$, among others.

In an embodiment, a data collection window and a sliding window, applied after the data collection window, are used when filtering the EDA signal. Preferably, the data collection window is comprised in a range between [0.5 and 8] seconds and the sliding window duration is of at least 1 second.

In an embodiment, the filtering is a low pass filtering. In this case, detail coefficients representing high frequency (> 0.5 Hz) of the signal (i.e. noise) are set to zero. Moreover, detail coefficients representing lower frequency (< 0.5 Hz) are modeled with Laplace distribution using the following equation: $\tilde{d}_j \sim Laplace(0,b)$, and the scale parameter $\hat{b}$ of the Laplace distribution is estimated for each level $j$ from the wavelet coefficients, using Maximum-likelihood estimation as per equation: $\hat{b} = \frac{1}{N}\sum_{i=1}^{N}|d_j^i|$. This modeling can be done before applying the low pass filtering or after applying the low pass filtering. Preferable, it is done after the low pass filtering.

Besides, a wavelet transform shrinking on detail coefficients according to the following equation:

$$\hat{d}_j^i = \begin{cases} \hat{d}_j^i & T_{low} \le \hat{d}_j^i \le T_{high} \\ 0 \; otherwise \end{cases},$$

can be also applied. Where the modelling and the wavelet transform shrinking are repeated on the wavelet coefficients of all higher levels and then inverse SWT wavelet transform is applied to the thresholded wavelet coefficients to obtain a fully denoised EDA signal.

In an embodiment, the calculated set of feature values may comprise event related features such as Skin Conductance Response (SCR), amplitude, Skin Conductance Level (SCL), number of peaks, peak amplitude, average rise time, etc.; statistical features such as mean, standard deviation, first/second order difference, kutosis, skewness, etc.; and power features such as energy, power spectral density and spectral power in different frequency bands, among others. Event related features are preferably analyzed according to previously established criteria, such as ones mentioned in [9, 10].

According to the proposed method, the classification of the subject's arousal and/or stress level may be performed either using a Support vector machine (SVM) classifier or using an Artificial Neural Network (ANN), or any other classification method.

In an embodiment, the obtained arousal and/or stress level is combined with a valence level corresponding to a behavior activation associated with emotion obtained from an EEG signal acquired from the subject.

In yet another embodiment, the obtained arousal and/or stress level besides being combined with the valence level is further combined with external information about the subject (e.g. eye tracking) acquired via an eye-tracking device. In this manner a three dimensional cognitive state, comprise of level of attention and emotional state of the subject is monitored and processed particularly on line.

Additional aspects described herein also relate to a method for filtering an EDA signal via wavelet and further modelling detail coefficients of the wavelet using a Laplace distribution. Preferably, the EDA signal is obtained in response to one or more stimulus presented to a subject during a given period of time using a measuring device.

Other aspects of the invention that are disclosed herein also include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

Therefore, present invention provides an efficient wavelet-based method for artifacts attenuation detection and correction of EDA signals in online, i.e. real time filtering, with low computational cost. Present invention allows measuring emotional state and engagement level (i.e. human emotion) of a subject in real time, being the capturing and processing of the signal up to 863 times faster than equivalent steps of the state of the art method.

Brief Description of the Drawings

The previous and other advantages and features will be more fully understood from the following detailed description of embodiments, with reference to the attached drawings, which must be considered in an illustrative and non-limiting manner, in which:

Fig. 1 is a flow diagram schematically illustrating the steps of a method for the measurement of human emotion of a subject, in accordance with an embodiment of the present invention.

Fig. 2 is a block diagram schematically illustrating the architecture used by the proposed method, in accordance with an embodiment of the present invention.

Fig. 3 is a graph showing results of the original EDA signal and after having been filtered.

Fig. 4 is a block diagram schematically showing the architecture used by the proposed method, in accordance with another embodiment of the present invention. As shown in figure, the present invention includes sensor units for detecting one or more physiological and/or eye-tracking data and sensor control units for acquiring and analyzing the physiological signals and/or eye-tracking data.

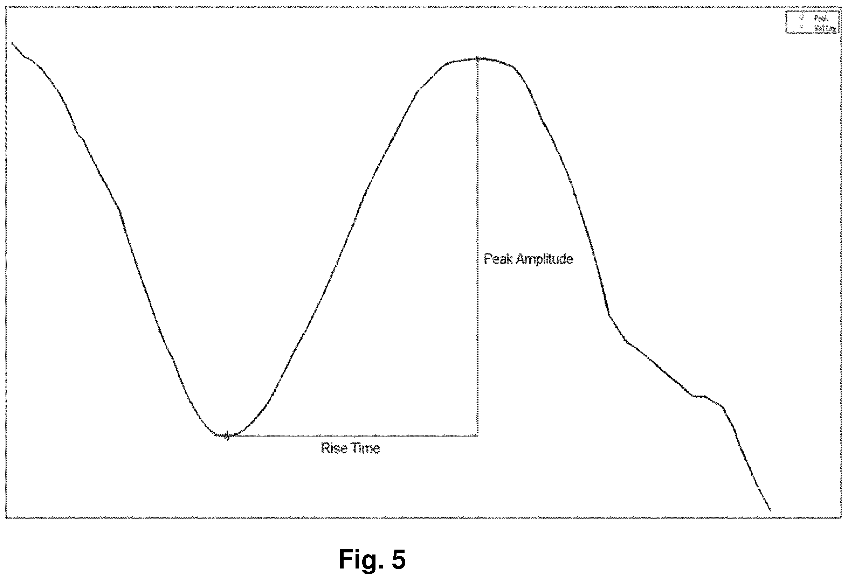

Fig. 5 is a graph showing waveform of the EDA segment used to detect Event -Related Features such as Peak Amplitude, Number of Peaks, Average Rising Time, etc.

Detailed Description of the Invention and of Preferred Embodiments

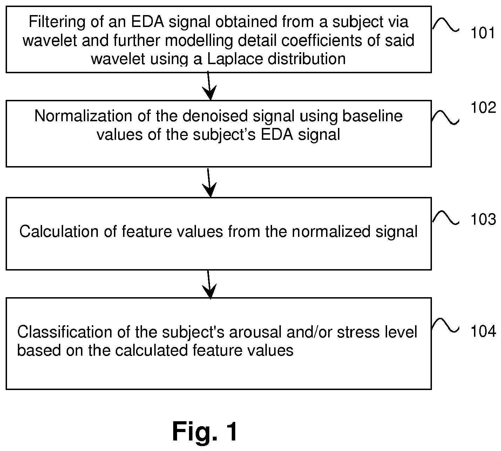

The present invention provides, in an embodiment, as disclosed in Fig. 1, a method, implemented through at least one software program running on at least one computing hardware device for online measurement of the arousal and/or stress level of a subject using his/her Electro-dermal Activity (EDA) (also his/her Galvanic Skin Response (GSR) could be used), comprising (step 101) filtering of the obtained EDA signal; normalization (step 102) of the denoised signal using the baseline values of the subject's EDA signal, for instance by subtracting the baseline minimum and dividing by the baseline range; calculation of the feature values (step 103) from the signal; and online classification of the subject's arousal and/or stress level (an index value) (step 104) using for instance a SVM or an ANN classifier on extracted features. Fig. 1 shows a schematic illustration of the main steps of the method. All the different steps are executed, preferably, online, i.e. in real time, this being possible just due to the particularities of the invention, in particular speed of processing, as previously stated.

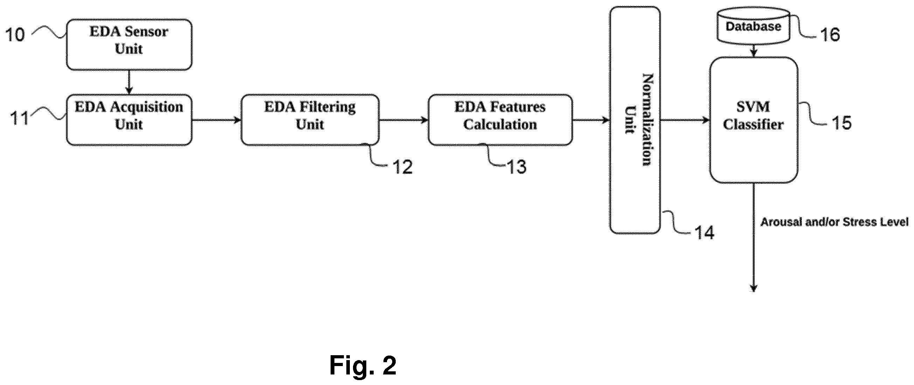

Fig. 2 illustrates a possible architecture used for implementing the above described method. It should be noted that the different steps of the method can be executed by a single processor or unit of the computing hardware device or by independent processors or units, each one being responsible of one particular task or step.

In an example embodiment, a data collection window of 8 seconds is selected for processing of the EDA signal, from now on only termed signal. After initial 8 seconds, a sliding window duration of 1 second is applied, so arousal and/or stress index is available every 1 second after first 8 seconds. The data collection window and sliding window duration are variable. The data collection window can vary from 0.5 to a value higher than 8 seconds. However, values higher than 8 seconds will be less useful.

A main source of artifacts is the power line noise resulting from the AC frequency input of 50 Hz (in Europe). Since the typical frequency of the EDA signal is 0.0167-0.25 Hz this type of artifact can be treated as high frequency noise and hence can be removed via filtering.

In the example embodiment, the filtering of the signal is performed by modeling the signal via Stationary Wavelet Transform (SWT) using *haar* as mother wavelet and level of decomposition *J* is set to ($log_2 freq$ + 2), where *freq* is the frequency of the signal. The signal frequency is selected to be a power of 2, while collecting the signal. If required, the signal can be up-sampled or down-sampled to obtain a frequency in power of 2. It should be noted that other wavelets could be used as mother wavelets, for example *db3* and *Coiflet3*. Detail coefficients representing high frequency (> 0.5 Hz) of the signal (i.e. noise) are set to zero, thereby low pass filtering the signal.

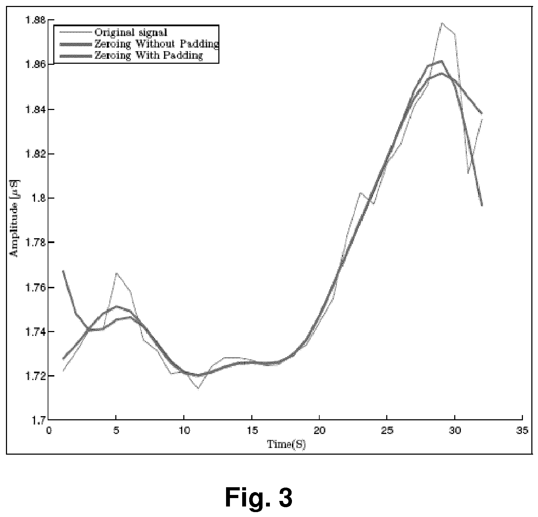

The low pass filtering distorts the signal at the beginning and at the end (Fig. 3). To get rid of the distortion, a padding can be applied at the beginning and at the end of the signal before low pass filtering. After applying the extension, the low pass filtering preserves the signal.

The most important source of physiologically based artifacts are motions (of the subject or of the part of the body of the subject where the signal by means of electrodes is acquired), which include skin movements beneath the electrodes acquiring the signal and muscular activity occurring in non-recording locations. To filter such artifacts, the highest level wavelet detail coefficients of the low pass filtered signal in the example embodiment are modeled with Laplace distribution (Equation 1) and the scale parameter $\hat{b}$ of the Laplace distribution is estimated for each level *j* from the wavelet coefficients $d_j^i$ of the original signal *d*, using Maximum-likelihood estimation as per equation 2.

$$\tilde{d}_j \sim Laplace(0, b) \qquad \text{(Eq. 1)}$$

$$\hat{b} = \frac{1}{N} \Sigma_{i=1}^{N} |d_j^i| \qquad \text{(Ec. 2)}$$

Likewise, the $T_{low}$ and $T_{high}$ thresholds of wavelet shrinkage can be calculated using equations 3 and 4. The proportion of motion artifacts in original signal can be determined by the approximation of the noise variance $\sigma^2$ in the signal. Preferably, the proposed method applies $\delta$= 0.10 but this may vary.

$$T_{low} = -T_{high} \qquad \text{(Ec. 3)}$$

$$T_{high} = \hat{b} * log_e(\delta) \qquad \text{(Ec. 4)}$$

Preferably, wavelet transform shrinking is applied on detail coefficients according to:

$$\hat{d}_j^i = \begin{cases} \hat{d}_j^i & T_{low} \leq \hat{d}_j^i \leq T_{high} \\ 0 & otherwise \end{cases} \qquad \text{(Ec. 5)}$$

The modelling and the wavelet transform shrinking are repeated on the wavelet coefficients of all higher levels and then inverse SWT wavelet transform is applied to the thresholded wavelet coefficients to obtain a fully denoised EDA signal.

In an embodiment, the baseline values are also denoised using the above described processes prior to normalization.

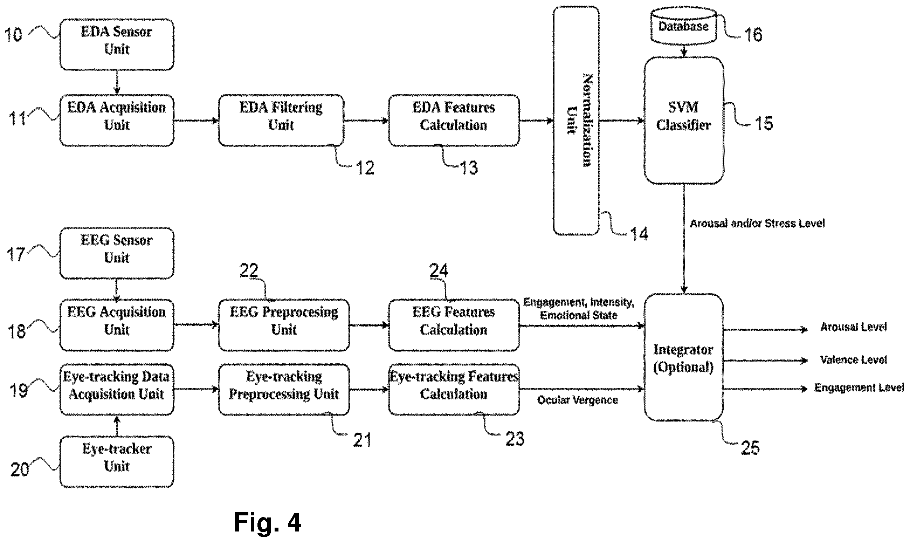

In another embodiment, the obtained arousal and/or stress level is combined with a valence level corresponding to a behavior activation associated with emotion. The valence level is preferably obtained from an EEG signal acquired from the subject. Therefore, the emotional state in terms of human emotion can be represented using a two-dimensional space. Moreover, the arousal and/or stress level and the valence level may be further combined with external information of the subject, e.g. eye tracking, acquired via an eye-tracking device configured to perform such action. Therefore, the emotional state in terms of human emotion will be represented using a comprehensive model comprising arousal, valence and level of engagement. Fig. 4 illustrates this particular embodiment. The present invention accordingly can measure and/or acquire EDA, EEG signals and eye-tracking movements in response to a stimulus in order to obtain the arousal level, valence and attention/engagement level of the user. To this end, the invention includes an EDA sensor unit 10 for acquiring EDA signals, an EEG sensor unit 17 for acquiring EEG activity and an eye-tracking unit (or device) 20 to capture the eye movements and related data. The acquired EDA signals 11 are then filtered via filtering unit 12 and feature calculation thereof 13 is performed as detailed before. The acquired EEG activity 18 and the eye-tracked data 19 are pre-processed 21, 22, and features thereof also calculated 23, 24.

It is preferred that the EDA and EEG sensors 10, 17 are wireless wearable devices and the eye-tracking unit 20 is a screen eye-tracker.

For example, the invention can monitor, online, the global cognitive state comprised of level of attention and emotional state of the user, as illustrated in Fig. 4, which can be understood as follows:

- Attention defines the mental ability to select stimuli, responses, memories, or thoughts that are behaviorally relevant, among the many others that are behaviorally irrelevant. Attention can be better monitored via eye-tracking technology. Eye tracking technology offers the possibility of capturing visual behavior in real time and monitoring locations of fixations on the monitor. Eye-tracking data will be captured preferably via screen-based eye trackers such as Tobii X60 eye tracker.

- Emotional state can be further reduced to two fundamental dimensions, Arousal and Valence:

    • Valence is the direction of the behavioral activation associated with emotion, either toward (appetitive motivation, pleasant emotion) or away from (aversive motivation, unpleasant emotion) a stimulus. Emotional Valence (motivation, frustration, cognitive workload etc.) is better understood by Electroencephalography (EEG) analysis. EEG is a well-established non-invasive technique for monitoring electrical activity generated by the brain with high temporal resolution and relatively low cost. The EEG is recorded throughout the stimuli presentation, preferably via wearable devices such as Emotiv Epoc.

    • Arousal is orthogonal to the valence and is the intensity of the emotional activation, ranging from excited to calm. EDA analysis as explained via description, offers online access to underlying changes in emotional arousal.

Therefore, a particular procedure for monitoring the global cognitive state can be:

1. The user is equipped with an EDA capturing device, or EDA sensor unit, 10 and an EEG headset, or EEG sensor unit 17, and an eye-tracker unit 20 is placed directly below a monitor at a 30° viewing angle to acquire participant's eye position.

2. A calibration is done with the eye-tracker unit 20 to ensure accuracy of both eyes to meet preferably a minimum of 0.5°.

3. The EDA signals acquisition unit 11, EEG signals acquisition unit 18 and the eye-tracker data acquisition unit 19 will run on the same computing hardware device such as a PC, thereby timestamps for all the data will use the same system clock while reporting the values.

4. EDA signal analysis will be done for the consecutive window duration (preferably 8 seconds).

5. The data captured via eye-tracker unit 20 preferably includes, 3D eye position, screen gaze position and pupil diameter and is analyzed as follows:

- From above eye-tracking data ocular eye-vergence is calculated over the next window duration (preferably 8 seconds), by transforming the X and Y coordinates of both the eyes in angular units through algorithms designed to calculate 3-D components of both eye gaze vectors, for instance using the methodology described in [7]. The transformation is performed taking into account the real distance of the screen to the observer and the actual inter-pupil distance.

- All the above procedure for eye-capturing data is performed online and the level of attention is represented via the ocular vergences for the whole window duration. Divergence often happens when our mind drifts away, when losing focus or concentration.

- The eye-tracking analysis is not limited to ocular vergence metric analysis only and may also contain extraction and analysis of other sophisticated eye-tracking metrics such as pupil size / dilation, distance to the screen etc.

6. Simultaneously, the average band powers from all available electrodes of EEG sensor unit 17 is collected. If the average band power is directly not available, raw EEG data is collected and average band power is calculated online for all electrodes. Then the average band power EEG data from all electrodes is analyzed as follows:

- Total average band powers are calculated for theta (3-7 Hz), alpha (8-12 Hz) and beta (13-30 Hz) bands from all available electrodes (14 in case of Emotiv Epoc). Engagement Index is calculated as [8]:

$$engagementIndex = (totalBeta/(totalAlpha + totalTheta)).$$

- Frontal Alpha Asymmetry can be calculated as per following equation:

$$frontalAlphaAssymetry = \log(leftFrontalAlpha) - \log(rightFrontalAlpha),$$

where leftFrontalAlpha and rightFrontalAlpha are frontal alpha band values, preferably using AF3 electrode for leftFrontalAlpha and AF4 electrode for rightFrontalAlpha. Using frontalAlphaAssymetry value emotional state can be classified as per following equation:

$$if(frontalAlphaAssymetry < threshold)$$

$$emotionalState = 'Positive'$$

$$else$$

$$emotionalState = 'Negative'$$

- The threshold value can be preferably calculated from the baseline values of the alpha band power of frontal electrodes (preferable AF3 and AF4) for the user.

$$Threshold = leftFrontalAlpha - rightFrontalAlpha$$

- Emotional Intensity is calculated as per following equations [8]:

$$frontalAlphaPower = \log(rightFrontalAlpha) + \log(leftFrontalAlpha) emotionalIntensity = 1/frontalAlphaPower$$

7. EDA, Eye-tracking and EEG derived metrics are used separately or can be combined using statistical methods such as using mean, etc.

8. The EDA, EEG and the eye-tracking signals are recorded for the normal emotional state of the user, in prior to commencing the recognition of the emotional state. From recorded data, relevant features are extracted and features and/or raw signals are used in normalization of the data during the recognition.

9. The SVM or ANN classifier is used for training and classification of the emotional state of the user based upon the normalized feature values of EDA. This training and classification can also be extended to EEG and Eye-tracking feature values.

The present invention also provides a method for filtering an EDA signal acquired from a subject. The proposed wavelet-based method for artifacts attenuation or removal of the EDA signal uses a SWT modeling of the wavelet detail coefficients as a Laplace distribution.

The proposed invention can be implemented on EDA recordings, i.e. the EDA signal can be stored in a memory or database previous being processed. For instance, in a particular embodiment the proposed methods were tested on EDA recordings from publicly available driver dataset collected during real-world driving, and containing a high number of motion artifacts.

While various disclosed embodiments have been described above, it should be understood that they have been presented by way of example only, and not limitation. Numerous changes to the subject matter disclosed herein can be made in accordance with this disclosure without departing from the scope of this disclosure.

As will be appreciated by one skilled in the art, the subject matter disclosed herein may be embodied as a system, method or computer program product. Accordingly, this disclosure can take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, this disclosure may take the form of a computer program product embodied in any tangible medium of expression having computer usable program code embodied in the medium.

Any combination of one or more computer usable or computer readable medium(s) may be utilized. The computer-usable or computer-readable medium may be, for example, but not limited to, an electronic, magnetic, optical, electromagnetic, infrared, or semiconductor system, apparatus, or device. More specific examples (a non-exhaustive list) of the computer-readable medium would include non-transitory media including the following: an electrical connection having one or more wires, a portable computer diskette, a hard disk, a random access memory (RAM), a read-only memory (ROM), an erasable programmable read-only memory (EPROM or Flash memory), a portable compact disc read-only memory (CDROM), an optical storage device, or a magnetic storage device.

The scope of the present invention is determined by the claims that follow.

References:

[1] Hernandez, J., Morris, R.R., Picard, R.W., 2011. Call Center Stress Recognition with Person-Specific Models. Springer Berlin Heidelberg, Berlin, Heidelberg. pp. 125-134.

[2] Ming-Zher Poh, Loddenkemper, T., Swenson, N.C., Goyal, S., Madsen, J.R., Picard, R.W., 2010. Continuous monitoring of electrodermal activity during epileptic seizures using a wearable sensor, IEEE. pp. 4415-4418.

[3] Junli, L., Yanqin, H., Ping, W., Gang, C., 2007. A novel method for the determination of the wavelet denoising threshold, in: 2007 1st International Conference on Bioinformatics and Biomedical Engineering, pp. 713-716.

[4] Molavi, B., Dumont, G.A., 2012. Wavelet-based motion artifact removal for functional near-infrared spectroscopy. Physiological Measurement 33, 259-270.

[5] Swangnetr, M., Kaber, D.B., 2013. Emotional state classification in patient-robot interaction using wavelet analysis and statistics-based feature selection. IEEE Transactions on Human-Machine Systems 43, 63-75.

[6] Chen,W., Jaques, N., Taylor, S., Sano, A., Fedor, S., Picard, R., 2015. Wavelet-based motion artifact removal for electrodermal activity. Conference Proceedings: Annual International Conference of the IEEE Engineering in Medicine and Biology Society 2015,6223-6.

[7] Sole Puig M, Perez Zapata L, Aznar-Casanova JA, Super H (2013) A Role of Eye Vergence in Covert Attention. PLoS ONE 8(1): e52955. doi:10.1371/journal.pone.0052955

[8] Timothy McMahan, Ian Parberry, Thomas D. Parsons, Evaluating Player Task Engagement and Arousal Using Electroencephalography, Procedia Manufacturing, Volume 3, 2015, Pages 2303-2310, ISSN 2351-9789.

[9] Dawson, M. E., Schell, A. M., & Filion, D. L. (2000). The electrodermal system.

[10] J. T. Cacioppo (Ed.), Handbook of psychophysiology. USA: Cambridge University Press.

## Claims

**1.** A computer-implemented method for the measurement of human emotion of a subject, wherein an Electrodermal Activity, EDA, signal is obtained in response to one or more stimulus presented to a subject during a given period of time, the method being **characterized in that** it comprises:

a) filtering the obtained EDA signal via wavelet and further modelling detail coefficients of said wavelet using a Laplace distribution, obtaining a denoised EDA signal;

b) normalizing said denoised EDA signal using baseline values of the subject's EDA signal;

c) calculating a set of feature values from the normalized EDA signal; and

d) classifying a subject's arousal and/or stress level as a component of a human emotion based on the calculated set of feature values.

2. The method of claim 1, wherein said wavelet comprises a Stationary Wavelet Transform, SWT, and a level of decomposition *J* is set to ($log_2 freq + 2$), where *freq* is the frequency of the signal.

3. The method of claim 2, comprising using at least a *haar* wavelet as mother wavelet.

4. The method of any of the previous claims, wherein a data collection window and a sliding window, applied after said data collection window, are used when filtering in step a).

5. The method of claim 4, wherein the data collection window being comprised in a range between 0.5 and 8 seconds and the sliding window duration being of at least 1 second.

6. The method of previous claims, wherein said filtering of step a) being a low pass filtering by setting to zero detail coefficients representing high frequency > 0.5 Hz of the EDA signal.

7. The method of claim 6, wherein detail coefficients of the EDA signal representing < 0.5 Hz frequency are modeled with the Laplace distribution using the following equation: $\tilde{d}_j \sim Laplace(0, b)$, and a scale parameter $\hat{b}$ of the Laplace distribution is estimated for each level *j* from the wavelet detail coefficients, using Maximum-likelihood estimation as per equation: $\hat{b} = \frac{1}{N} \sum_{i=1}^{N} |d_j^i|$

8. The method of claim 7, further comprising applying a wavelet transform shrinking on detail coefficients according to the following equation:

$$\hat{d}_j^i = \begin{cases} \hat{d}_j^i & T_{low} \leq \hat{d}_j^i \leq T_{high} \\ 0 \ otherwise \end{cases},$$

and wherein said modelling and said wavelet transform shrinking are repeated on the wavelet coefficients of all higher levels and then inverse SWT wavelet transform is applied to the thresholded wavelet coefficients to obtain a fully denoised EDA signal.

9. The method of claim 1, wherein the calculated set of feature values of step c) comprises:

- event related features including a Skin Conductance Response, SCR, amplitude, a Skin Conductance Level, SCL, number of peaks, peak amplitude and average rise time;

- statistical features including mean, standard deviation, first/second order difference, kutosis and skewness; and

- power features including energy, power spectral density and spectral power in different frequency bands.

10. The method of any of the previous claims, wherein said classifying of step d) comprising using a classifier at least including a Support Vector Machine, SVM, or an Artificial Neural Network.

11. The method of claim 1, wherein said EDA signal being acquired by a wearable or non-wearable device.

12. The method of claim 1, further comprising combining the obtained arousal and/or stress level with a valence level corresponding to a behavior activation associated with emotion, wherein said valence level being obtained from an EEG signal acquired from the subject.

13. The method of claim 1, further comprising combining the obtained arousal and/or stress level with:

a valence level corresponding to a behavior activation associated with emotion, and

external information about the subject including eye tracking,

wherein said valence level being obtained from an EEG signal acquired from the subject and said external information being acquired via an eye-tracking device.

14. The method of any of the previous claims, wherein steps a) to d) are performed online, so the component of human emotion is directly obtained during the interaction of the subject with information and communication applications.

15. A computer-implemented method for filtering an electrodermal activity, EDA, signal, wherein said EDA signal being obtained in response to one or more stimulus presented to a subject during a given period of time, using a measuring device, **characterized in that** the method comprises performing said filtering via wavelet and further modelling detail coefficients of said wavelet using a Laplace distribution.

Filtering of an EDA signal obtained from a subject via wavelet and further modelling detail coefficients of said wavelet using a Laplace distribution | 101

Normalization of the denoised signal using baseline values of the subject's EDA signal | 102

Calculation of feature values from the normalized signal | 103

Classification of the subject's arousal and/or stress level based on the calculated feature values | 104

# Fig. 1

**Fig. 2**

EP 3 466 322 A1

**Fig. 3**

Fig. 4

Fig. 5

Europäisches
Patentamt

European
Patent Office

Office européen
des brevets

**EUROPEAN SEARCH REPORT**

Application Number

EP 17 38 2661

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MANIDA SWANGNETR ET AL: "Emotional State Classification in Patient Robot Interaction Using Wavelet Analysis and Statistics-Based Feature Selection", IEEE TRANSACTIONS ON HUMAN-MACHINE SYSTEMS, IEEE, PISCATAWAY, NJ, USA, vol. 43, no. 1, 1 January 2013 (2013-01-01), pages 63-75, XP011484268, ISSN: 2168-2291, DOI: 10.1109/TSMCA.2012.2210408 * abstract * * B. Physiological Measures of Emotional States; page 64 * * B. Data Post-Processing and Overall Results; page 67 - page 68 * * A. Event Selection and Analytical Procedure; page 68 * * C. Physiological Feature Extraction: GSR Analysis; page 69 - page 71; figures 7, 9 * * D. Feature Selection; page 71 * * E. Emotional State Classification; page 71 - page 72 *<br><br>-----<br><br>-/-- | 1-15 | INV.<br>A61B5/00<br>A61B5/053<br>A61B5/16<br><br><br>TECHNICAL FIELDS SEARCHED (IPC)<br><br>A61B<br>G06F |

The present search report has been drawn up for all claims

| Place of search | Date of completion of the search | Examiner |
|---|---|---|
| The Hague | 6 April 2018 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 17 38 2661

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Shanbin Zhang ET AL: "Classification of Evoked Emotions Using an Artificial Neural Network Based on Single, Short-Term Physiological Signals", Journal of Advanced Computational Intelligence and Intelligent Informatics, 20 January 2015 (2015-01-20), pages 118-126, XP055428927, DOI: 10.20965/jaciii.2015.p0118 Retrieved from the Internet: URL:http://or.nsfc.gov.cn/bitstream/000019 03-5/328139/1/1000013718421.pdf [retrieved on 2017-11-27] * abstract * * 2. Signals and Features; page 119 * * GSR signals de-noising; page 120 * * 2.2. Features Set; page 120 - page 121 * * 3. The ANN Classifier Model; page 121 - page 122; figure 3 * * 4. Emotion Classification; page 122 - page 123 * * tables 3,4 * * 5. Conclusions & Future Work; page 123 - page 124 * ----- -/-- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 6 April 2018 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JAINENDRA SHUKLAEMAIL ET AL: "MuDERI: Multimodal Database for Emotion Recognition Among Intellectually Disabled Individuals", NETWORK AND PARALLEL COMPUTING, vol. 9979, 7 October 2016 (2016-10-07), pages 264-273, XP055427978, Cham ISSN: 0302-9743 ISBN: 978-3-642-35622-3 * the whole document * | 1-15 | |
| A | MARCO PEDROTTI ET AL: "Automatic Stress Classification With Pupil Diameter Analysis", INTERNATIONAL JOURNAL OF HUMAN-COMPUTER INTERACTION., vol. 30, no. 3, 31 January 2014 (2014-01-31), pages 220-236, XP055429354, US ISSN: 1044-7318, DOI: 10.1080/10447318.2013.848320 * the whole document * | 1-15 | |

TECHNICAL FIELDS SEARCHED (IPC)

The present search report has been drawn up for all claims

| Place of search | Date of completion of the search | Examiner |
|---|---|---|
| The Hague | 6 April 2018 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HERNANDEZ, J. ; MORRIS, R.R. ; PICARD, R.W.** Call Center Stress Recognition with Person-Specific Models. Springer Berlin Heidelberg, 2011, 125-134
- **MING-ZHER POH ; LODDENKEMPER, T. ; SWENSON, N.C. ; GOYAL, S. ; MADSEN, J.R. ; PICARD, R.W.** Continuous monitoring of electrodermal activity during epileptic seizures using a wearable sensor. IEEE, 2010, 4415-4418
- **JUNLI, L. ; YANQIN, H. ; PING, W. ; GANG, C.** A novel method for the determination of the wavelet denoising threshold. *2007 1st International Conference on Bioinformatics and Biomedical Engineering,* 2007, 713-716
- **MOLAVI, B. ; DUMONT, G.A.** Wavelet-based motion artifact removal for functional near-infrared spectroscopy. *Physiological Measurement,* 2012, vol. 33, 259-270
- **SWANGNETR, M. ; KABER, D.B.** Emotional state classification in patient-robot interaction using wavelet analysis and statistics-based feature selection. *IEEE Transactions on Human-Machine Systems,* 2013, vol. 43, 63-75
- **CHEN,W. ; JAQUES, N. ; TAYLOR, S. ; SANO, A. ; FEDOR, S. ; PICARD, R.** Wavelet-based motion artifact removal for electrodermal activity. *Conference Proceedings: Annual International Conference of the IEEE Engineering in Medicine and Biology Society 2015,* 2015, 6223-6
- **SOLE PUIG M ; PEREZ ZAPATA L ; AZNAR-CASANOVA JA ; SUPER H.** A Role of Eye Vergence in Covert Attention. *PLoS ONE,* 2013, vol. 8 (1), e52955
- **TIMOTHY MCMAHAN ; IAN PARBERRY ; THOMAS D. PARSONS.** Evaluating Player Task Engagement and Arousal Using Electroencephalography. *Procedia Manufacturing,* 2015, vol. 3, ISSN 2351-9789, 2303-2310
- **DAWSON, M. E. ; SCHELL, A. M. ; FILION, D. L.** *The electrodermal system,* 2000
- Handbook of psychophysiology. Cambridge University Press